# EUROPÄISCHE PATENTSCHRIFT

(11) **EP 4 489 677 B1**
(45) Veröffentlichungstag und Bekanntmachung des Hinweises auf die Patenterteilung: **21.10.2026**
(21) Anmeldenummer: 23710328.8
(22) Anmeldetag: 09.03.2023
(51) Int. Cl.: A61B 18/26, G02B 6/42, G02B 6/36

(54) **VORRICHTUNG ZUM ANSCHLIESSEN EINER APPLIKATIONSFASER ZUM APPLIZIEREN VON LASERLICHT FÜR CHIRURGISCHE ANWENDUNGEN AN EINEN LASER UND LASERAPPARATUR MIT EINEM LASER UND EINER SOLCHEN APPLIKATIONSFASER**
DEVICE FOR CONNECTING AN APPLICATION FIBRE, FOR APPLYING LASER LIGHT FOR SURGICAL USES, TO A LASER, AND LASER APPARATUS HAVING A LASER AND SUCH AN APPLICATION FIBRE
DISPOSITIF DE CONNEXION D'UNE FIBRE D'APPLICATION À UN LASER POUR L'APPLICATION DE LA LUMIÈRE LASER À DES FINS CHIRURGICALES, ET APPAREIL LASER COMPORTANT UN LASER ET UNE TELLE FIBRE D'APPLICATION

(30) Priorität: 09.03.2022 DE 102022105499
(43) Veröffentlichungstag der Anmeldung: 15.01.2025
(73) Patentinhaber: Futonics Laser GmbH, 37191 Katlenburg-Lindau (DE)
(72) Erfinder: FUHRBERG, Peter, 37077 Göttingen (DE)
(74) Vertreter: REHBERG HÜPPE + PARTNER
(86) Internationale Anmeldenummer: PCT/EP2023/055973
(87) Internationale Veröffentlichungsnummer: WO 2023/170193

(56) Entgegenhaltungen:
- CN-C- 100 337 138
- US-A1- 2002 114 566
- US-A1- 2013 148 925

## Beschreibung

### TECHNISCHES GEBIET DER ERFINDUNG

Die Erfindung bezieht sich auf eine Vorrichtung zum Anschließen einer Applikationsfaser zum Applizieren von Laserlicht für chirurgische Anwendungen an einen Laser. Insbesondere bezieht sich die Erfindung auf eine solche Vorrichtung mit den Merkmalen des Oberbegriffs des unabhängigen Patentanspruchs 1. Weiterhin bezieht sich die Erfindung auf eine Laserapparatur mit einem Laser und einer Applikationsfaser zum Applizieren von Laserlicht für chirurgische Anwendungen. Eine solche Laserapparatur kann beispielsweise zum Zerkleinern von wasserhaltigen Steinen, wie beispielsweise Nierensteinen, in Körperhöhlen verwendet werden. Insbesondere bezieht sich die Erfindung auf eine solche Laserapparatur mit den Merkmalen des Oberbegriffs des unabhängigen Patentanspruchs 11.

Beispielsweise zum Zerkleinern von wasserhaltigen Steinen in Körperhöhlen mit Hilfe von Laserlicht wird das von einem Laser bereitgestellte Laserlicht mit einer Applikationsfaser, d. h. einer optischen Faser, an den Ort seiner Applikation geleitet. In dem gewählten Beispiel wirkt das aus der Applikationsfaser austretende Laserlicht in der Körperhöhle auf den jeweiligen wasserhaltigen Stein ein, um ihn lokal so zu erhitzen, dass er in kleinere Teile zerfällt. Die Applikationsfaser wird bei dieser Verwendung insbesondere an ihrem distalen Faserende, aus dem das Laserlicht austritt, beansprucht und verunreinigt. Nach einiger Zeit Ihrer Verwendung ist sie soweit verbraucht, das sie ausgetauscht werden muss. Daher sind Vorrichtungen zum Anschließen einer Applikationsfaser an einen Laser üblich, die ein schnelles Auswechseln der Applikationsfaser ermöglichen.

### STAND DER TECHNIK

Zum Zerkleinern von Nierensteinen im Sinne eines Fragmentierens ist der Einsatz eines Holmium:YAG-Lasers bekannt, dessen Laserlicht über eine durch den Harnleiter geführte Applikationsfaser direkt bis an den jeweiligen Nierenstein herangeführt wird. Das aus der Applikationsfaser austretende Laserlicht des Holmium:YAG-Lasers mit einer Wellenlänge von 2.100 nm erhitzt den Nierenstein insgesamt oder lokal so, dass er durch verdampfende Wasseranteile fragmentiert wird. Um dies sicher zu erreichen, ist der Einsatz von Holmium:YAG-Lasern mit einer maximalen Spitzenleistung von 15 kW üblich. Derartige Holmium:YAG-Laser, insbesondere solche, die Pulswiederholraten von mehr als 30 Hz erreichen, sind aufwändig herzustellen. Zudem ist das Risikopotential des mit dieser maximalen Leistung austretenden Laserlichts groß, wenn das Laserlicht nicht nur wie geplant auf den Nierenstein, sondern auf angrenzendes Nierengewebe trifft.

Beim Fragmentieren von Nierensteinen mit Laserlicht von einem Holmium:YAG-Laser mit einem Laserkopf wird das Laserlicht typischerweise in Pulsen mit einer Pulsenergie größer einem Joule und einer Pulsfrequenz kleiner als 30 Hz appliziert. sind Holmium:YAG-Laser mit bis zu vier Laserköpfen und insgesamt einer mittleren Leistung bis 120 W zum Fragmentieren von Nierensteinen bekannt. Die dazu berichteten größten praktischen Fragmentierungsraten liegen typischer Weise bei 6,6 mg Nierenstein pro Sekunde bei 120 W mittlerer Leistung.

Zum Zerkleinern von Nierensteinen im Sinne eines Zerstäubens ist der Einsatz eines Thulium-Faserlasers zu beaufschlagen. Dessen Laserlicht mit einer Wellenlänge von 1.940 nm wird von Wasser um mehr als einen Faktor 2 stärker absorbiert als Laserlicht mit einer Wellenlänge von 2.100 nm. Ein Thulium-Faserlasers ist wegen seiner wesentlich besseren Strahlqualität in Verbindung mit dünneren und flexibleren Applikationsfasern einsetzbar als ein Holmium:YAG-Laser. Außerdem sind Thulium-Faserlaser kompakter, günstiger als Mehrkopflaser und langlebiger als Holmium:YAG-Laser. Dabei ist die um einen Faktor von mehr als 10 größere Energieeffizienz eines Thulium-Faserlaser ein wesentlicher Faktor.

Beim Zerstäuben von Nierensteinen mit Laserlicht von einem Thulium-Faserlaser wird das Laserlicht typischerweise in Pulsen niedriger Pulsenergie um 0,1 Joule mit einer hohen Pulswiederholrate von typischerweise einigen 100 Hz appliziert. Die dabei praktisch erreichten maximalen Zerkleinerungsraten liegen bei 3 mg Nierenstein pro Sekunde.

Wenn Nierensteine wahlweise zerstäubt und fragmentiert werden sollen, findet aktuell sowohl ein Thulium-Faserlaser mit daran angeschlossener spezieller Applikationsfaser als auch ein Holmium:YAG-Laser mit daran angeschlossener spezieller Applikationsfaser Verwendung.

Aus der DE 10 2016 118 663 B3 ist ein System zur chirurgischen, endovenösen Lasertherapie mit einem Lasergerät und einem Applikationsmodul bekannt. Das Lasergerät weist eine Laserlichtquelle mit einem ersten Laserdiodenelement auf und ist mit der Laserlichtquelle optisch verbunden. Das erste Laserdiodenelement weist eine Halbleiterschicht aus einer Antimon-Verbindung auf und ist so konfiguriert, dass Laserlicht mit einer Wellenlänge zwischen 1.800 nm und 2.000 nm erzeugbar ist. Das Applikationsmodul ist als biegeflexibler Katheter mit einem Lichtwellenleiter ausgebildet. Eine Steuerung des Lasergeräts ist derart konfiguriert, dass das Laserlicht kontinuierlich emittiert wird und an einem distalen Ende des Lichtwellenleiters eine Laserlichtleistung von höchstens 10 W aufweist. Das Lasergerät umfasst weiterhin eine Hilfslaserlichtquelle und einen Lichtsensor. Die Hilfslaserlichtquelle und der Lichtsensor sind mit der Steuerung derart gekoppelt, dass eine Laserlichtintensität der Laserlichtquelle in Abhängigkeit von einer spezifischen Fluoreszenzlichtsignatur des zu behandelnden Gewebes eingestellt wird.

Aus der US 2020 / 0 306 878 A1 ist eine Laserschweißvorrichtung bekannt. Die Laserschweißvorrichtung weist eine erste Lasereinrichtung auf, die einen ersten Laserstrahl in eine erste Lichtleiterfaser einkoppelt, und eine zweite Lasereinrichtung, die einen zweiten Laserstrahl in eine zweite Lichtleiterfaser einkoppelt. Weiterhin sind Mittel vorhanden, um einen kombinierten Laserstrahl aus dem aus der jeweiligen Lichtleiterfaser wieder austretenden ersten Laserstrahl und zweiten Laserstrahl auszubilden. Der austretende erste Laserstrahl hat einen kreisförmigen Querschnitt und der austretende zweite Laserstrahl hat eine Ringform, die konzentrisch zu dem austretenden ersten Laserstrahl ist. Die Mittel zum Ausbilden des kombinierten Laserstrahls weisen eine Lichtleiterfaser mit zwei konzentrisch angeordneten Kernen und ein mehrlöchriges Kapillarrohr, in das auf der einen Seite die Lichtleiterfasern von der ersten und der zweiten Lasereinrichtung einmünden und aus dem auf der anderen Seite die Lichtleiterfaser mit den zwei Kernen austritt.

Aus der DE 10 2020 116 268 A1 sind eine Vorrichtung und ein Verfahren zur Veränderung des Strahlparameterprodukts bei Diodenlasern im Rahmen der Materialbearbeitung bekannt. Bei der Lasermaterialbearbeitung, insbesondere zum Schneiden oder für remote Schweißanwendungen werden Laser mit einem guten Strahlparameterprodukt verwendet. Singlemode-Laser im Wellenlängenbereich von 1 µm haben jedoch einen schlechten Schneidwirkungsgrad, wenn es um das Schneiden von mittleren oder dicken Materialien geht. So erzeugt ein so genanntes Monomode-Strahlprofil beim Schneiden von Material nur einen sehr dünnen Schnitt, der nicht breit genug ist, um das geschmolzene Material bei dickeren Materialien auszuwerfen. Die Vorrichtung zur Lasermaterialbearbeitung umfasst Laserdioden als Laserquelle, eine Fokussierlinse, eine Faser, in die das Laserlicht von den Laserdioden eingekoppelt wird, wobei das Strahlparameterprodukt der Faser größer ist als das Strahlparameterprodukt des einfallenden Laserlichts, und ein Substrat zur Erzeugung eines Versatzes zur Strahlachse. Das Verfahren zielt auf eine Erhöhung der Kopplungsdivergenz zur Abnahme des Strahlparameterprodukts ab. Es umfasst das Erzeugen eines Laserstrahls mittels eines Diodenlasers, das Fokussieren des Laserstrahls mittels einer Fokussierlinse und das Einkoppeln des Laserstrahls in eine Faser, wobei die Divergenz, Punktgröße oder der Einfallswinkel des Laserstrahls mittels eines Substrats verändert wird. Die Divergenz oder Punktgröße oder der Einfallswinkel des Strahls werden am Fasereingang verändert, um eine echte Änderung des Strahlparameterprodukts innerhalb der Grenzen der Faser oder einen Ring im Kern zu erzeugen. Durch Verwendung einer Faser mit einem größeren Akzeptanzwinkel (NA) als dem des einfallenden Laserstrahls kann das Strahlparameterprodukt am Faserausgang dadurch verringert werden, dass die Einfallswinkeldivergenz oder der Einfallswinkel an der Eingangsseite erhöht werden. Ein Donut-Profil als Spotgeometrie des aus der Faser austretenden Laserstrahls entsteht, wenn die ankommende Divergenz viel kleiner als der Akzeptanzwinkel der Faser ist, und die Fleckgröße viel kleiner als die Kerngröße ist. Das minimale Strahlparameterprodukt wird durch die grundlegenden Eigenschaften des Diodenlasers definiert und ist daher niedrig genug, um dünne Materialien sehr gut zu schneiden. Durch die gezielte Veränderung des Strahlparameterprodukts kanneine Anpassung an andere Materialien mit optimierter Strahlcharakteristik in Bezug auf das Strahlparameterprodukt oder Spot-Donut-Profil vorgenommen werden.

Aus der EP 1 773 534 B2 sind ein Verfahren zum Betrieb einer Lasereinrichtung und eine Lasereinrichtung mit einem Laserwerkzeug bekannt, das seinen Arbeitsabstand gegenüber einem Werkstück in einem Stellbereich ändern kann. Das Laserwerkzeug weist eine Laseroptik mit einer Fokussier- und einer Kollimationsoptik sowie einen Anschluss für einen Lichtwellenleiter mit einem Leiterauskopplungspunkt auf. Die Brennweite der Fokussieroptik wird durch Verstellen des optischen Abstands des Leiterauskopplungspunkts von der Kollimationsoptik verändert, wobei die Kollimationsoptik in Strahlrichtung mittels einer motorisch angetriebenen Stelleinrichtung fernsteuerbar verschoben wird. Die Stelleinrichtung ist mit der Steuerung eines mehrachsigen Manipulators verbunden, der das Laserwerkzeug oder das Werkstück führt, wobei die Bewegungen der Stelleinrichtung und des Manipulators miteinander verknüpft sind.

Aus der US 2002 / 0 114 566 A1 ist eine optische Einrichtung zur Verwendung einer optischen Faser bekannt, deren Endfläche nicht in einer rechtwinklig zu ihrer Faserachse ausgerichteten Ebene verläuft, sondern unter einem Neigungswinkel von beispielsweise 8° zu einer solchen Ebene. Die Auswirkung dieses Neigungswinkels auf die Richtung von in die optische Faser eingekoppeltem Licht wird durch einen Winkel zwischen der Faserachse und einer mittleren Ausbreitungsrichtung des Lichts vor der Endfläche, in dem Beispiel mit einer kommerziellen Quarzfaser durch einen Winkel von 3,4°, kompensiert, so dass sich das Licht in der Faser mit einer mittleren Ausbreitungsrichtung längs der Faserachse ausbreitet.

### AUFGABE DER ERFINDUNG

Der Erfindung liegt die Aufgabe zugrunde, eine Vorrichtung zum Anschließen einer Applikationsfaser an einen Laser und eine Laserapparatur mit einem Laser und einer Applikationsfaser aufzuzeigen, mit denen das Laserlicht in besonders effizienter Weise beispielsweise zum Zerkleinern von wasserhaltigen Steinen in Körperhöhlen appliziert wird.

### LÖSUNG

Die Aufgabe der Erfindung wird durch eine Vorrichtung mit den Merkmalen des unabhängigen Patentanspruchs 1 und eine Laserapparatur mit den Merkmalen des unabhängigen Patentanspruchs 11 gelöst. Bevorzugte Ausführungsformen der Vorrichtung und der Laserapparatur sind in den abhängigen Patentansprüchen definiert.

### BESCHREIBUNG DER ERFINDUNG

Eine erfindungsgemäße Vorrichtung dient zum Anschließen einer Applikationsfaser zum Applizieren von Laserlicht für chirurgische Anwendungen, die einen quer zu einer Faserachse verlaufenden Lichteintrittsquerschnitt aufweist, an einen Laser, der das Laserlicht an einem Lichtausgabeort mit einer mittleren Lichtausbreitungsrichtung ausgibt. Die Vorrichtung weist einen Laseranschluss zum Anschließen des Lasers, einen Faseranschluss zum Anschließen der Applikationsfaser und eine relativ zu dem Laseranschluss und dem Faseranschluss angeordnete Fokussieroptik auf. Der Laseranschluss richtet den Lichtausgabeort und die mittlere Lichtausbreitungsrichtung des Laserlichts gegenüber der Vorrichtung aus. Der Faseranschluss richtet die Faserachse und den Lichteintrittsquerschnitt der Applikationsfaser gegenüber der Vorrichtung aus. Die Fokussieroptik ist derart angeordnet, dass sie einen Teil des von dem an den Laseranschluss angeschlossenen Laser an dem Lichtausgabeort ausgegebenen Laserlichts durch deren Lichteintrittsquerschnitt in die an den Faseranschluss angeschlossene Applikationsfaser einkoppelt und dazu mit einem Konvergenzvollwinkel von nicht mehr als 2° in den Lichteintrittsquerschnitt fokussiert.

Zudem koppelt die Fokussieroptik den Teil des an dem Lichtausgabeort ausgegebenen Laserlichts unter einem Winkel zwischen seiner mittleren Lichtausbreitungsrichtung und der Faserachse im Bereich von 2° bis 10° in die Applikationsfaser ein und/oder der Faseranschluss ist derart verschwenkbar, dass der Winkel zwischen der mittleren Lichtausbreitungsrichtung des in die Applikationsfaser eingekoppelten Laserlichts und der Faserachse in einem Bereich von 0° bis mindestens 5° verstellbar ist.

Dass der Lichteintrittsquerschnitt quer zu der Faserachse der Applikationsfaser verläuft, kann - muss aber nicht - bedeuten, dass die Faserachse normal zu dem Lichteintrittsquerschnitt verläuft. Wenn die Faserachse nicht normal zu dem Lichteintrittsquerschnitt verläuft, sind die Richtung, in der der Lichteintrittsquerschnitt gegenüber der Faserachse geneigt ist, die Größe dieser Neigung und die daraus resultierende Ablenkung des Laserlichts bei seinem Einkoppeln in die Applikationsfaser zu berücksichtigen.

Bei der erfindungsgemäßen Vorrichtung ist es nicht wesentlich, dass die Fokussieroptik einen gegenüber dem gesamten an dem Lichtausgabeort ausgegebenen Laserlicht reduzierten Teil in die Applikationsfaser einkoppelt und dazu in den Lichteintrittsquerschnitt fokussiert. Regelmäßig wird jedoch nicht das gesamte von dem Laser ausgegebene Laserlicht in die Applikationsfaser eingekoppelt werden, schon allein, weil die Fokussieroptik keine Transmission von 100 % aufweist und auch an dem Lichteintrittsquerschnitt ein Anteil des Laserlichts reflektiert wird.

Mit der erfindungsgemäßen Vorrichtung wird das Laserlicht unter Ausnutzung nur eines kleinen Teils einer numerischen Apertur der Applikationsfaser, die typischerweise bei NA=0,22 oder darüber liegt, in die Applikationsfaser eingekoppelt. Entsprechend tritt das Laserlicht am Ort seiner gewünschten Anwendung auch nur über einen kleinen Teilbereich der numerischen Apertur der Applikationsfaser aus. Konkret wird typischerweise von nicht mehr als einem Zehntel der numerischen Apertur der Applikationsfaser beim Einkoppeln des Laserlichts durch den Lichteintrittsquerschnitt Gebrauch gemacht.

Erreicht werden kann der kleine Konvergenzvollwinkel des in den Lichteintrittsquerschnitt fokussierten Laserlichts dadurch, dass die Fokussieroptik eine Kollimierlinse und eine Fokussierlinse aufweist, wobei die Fokussierlinse eine um mindestens einen Faktor 5, vorzugsweise um mindestens einen Faktor 8 und beispielsweise um etwa einen Faktor 10 größere Brennweite aufweist als die Kollimierlinse. Wenn das von dem Laser an dem Lichtabgabeort abgegebene Laserlicht über einen größeren Divergenzvollwinkel, d. h. eine größere numerische Apertur des Lasers, verteilt ist, kann auch noch ein größerer Faktor zwischen den Brennweiten erforderlich oder zumindest sinnvoll sein.

Vorzugsweise weist die Fokussierlinse der Fokussieroptik der erfindungsgemäßen Vorrichtung eine Brennweite von mindestens 10 cm auf. Noch mehr bevorzugt liegt die Brennweite in einem Bereich zwischen 12 cm und 20 cm. Durch die lange Brennweite der Fokussierlinse wird bei gleichem Divergenzvollwinkel des in die Vorrichtung eintretenden Laserlichts ein kleinerer Konvergenzwinkel des in den Lichteintrittsquerschnitt fokussierten Laserlichts realisiert und damit eine besonders hohe Konzentration der Intensität des wieder aus der Applikationsfaser austretenden Laserlichts.

In einer besonders einfachen und zugleich bevorzugten Ausführungsform weist die Fokussieroptik nur die Fokussierlinse, d. h. eine einzige konvexe Linse auf.

Der Fokussieroptik kann eine den Konvergenzvollwinkel begrenzende Aperturblende der Vorrichtung zugeordnet sein. Außerhalb des gewünschten Konvergenzvollwinkels verlaufendes Laserlicht ist bei der Verwendung der erfindungsgemäßen Vorrichtung aber grundsätzlich nicht störend, solange es in den Lichteintrittsquerschnitt der Applikationsfaser fokussiert wird. Die Funktion der Aperturblende ist es, dass zurück zu dem Laseranschluss reflektiertes Laserlicht den Laseranschluss nicht beschädigt.

In der einen Ausführungsform der erfindungsgemäßen Vorrichtung koppelt die Fokussieroptik den Teil des an dem Lichtausgabeort ausgegebenen Laserlichts unter einem Winkel zwischen seiner mittleren Lichtausbreitungsrichtung und der Faserachse im Bereich von 2° bis 10° in die Applikationsfaser ein. Damit wird das Laserlicht in eine an den Faseranschluss als Applikationsfaser angeschlossene Multimode-Faser so eingekoppelt, dass in der Applikationsfaser eine Ringmode angeregt wird, die auch als Donut-Mode bezeichnet wird. Das Einkoppeln des Laserlichts unter dem Winkel zwischen seiner mittleren Lichtausbreitungsrichtung und der Faserachse der Applikationsfaser kann durch einen Winkel zwischen der Faserachse und der mittleren Lichtausbreitungsrichtung des in den Lichteintrittsquerschnitt fokussierten Laserlichts und/oder eine Neigung des Lichteintrittsquerschnitts gegenüber der Faserachse erreicht werden.

Bei der in der Applikationsfaser angeregten Ringmode konzentriert sich das elektromagnetische Feld des Laserlichts auf einen zu der Faserachse koaxial angeordneten Hohlzylinder. Das aus einem normal zu der Faserachse ausgerichteten Lichtaustrittsquerschnitt am distalen Ende der Applikationsfaser austretende Laserlicht breitet sich dann nicht über einen Vollkegel, sondern über einen Hohlkegel aus, bei dem der Bereich höchster Lichtintensität sowohl nach außen als auch nach innen durch einen Kegelmantel begrenzt ist. Dabei ist ein Vollwinkel des inneren Kegelmantels um den Konvergenzwinkel des in den Lichteintrittsquerschnitt fokussierten Laserlichts kleiner als der doppelte Winkel zwischen der mittleren Lichtausbreitungsrichtung des Laserlichts und der Faserachse, während ein Vollwinkel des äußeren Kegelmantels um den Konvergenzwinkel größer als dieser doppelte Winkel ist. Somit verteilt sich die Intensität des aus der Applikationsfaser austretenden Laserlichts nur über einen kleinen Teil der numerischen Apertur der Applikationsfaser und damit über nur einen kleinen Teil des Bereichs, über den sich die Intensität von Laserlicht verteilen würde, das über die gesamte numerische Apertur der Applikationsfaser hinweg in die Applikationsfaser eingekoppelt würde, deren numerische Apertur typischerweise bei NA = 0,22 liegt, was einem Aperturvollwinkel von 25° entspricht.

Durch die starke Konzentration des Laserlichts auf das kleine Volumen des Hohlkegels wird über dieses Volumen eine sehr hohe Intensität des aus der Applikationsfaser austretenden Laserlichts erreicht, und auch die räumliche Verteilung dieser hohen Intensität, d. h. der Hohlkegel, scheint für das Zerkleinern von wasserhaltigen Steinen sehr günstig zu sein. Jedenfalls sind mit Hilfe der erfindungsgemäßen Vorrichtung hohe Zerkleinerungsraten bei wasserhaltigen Steinen erreichbar, wie noch näher erläutert werden wird. Diese hohe Zerkleinerungsraten beruhen mutmaßlich auch darauf, dass die Ringmode in dem jeweiligen Stein ein Flat Top-Temperaturprofil erzeugt. Dadurch wird die applizierte Lichtenergie optimal für den Verdampfungsprozess des Wassers in dem Stein genutzt, weil das Wasser in einem maximal möglichen Volumen verdampft wird.

Die mit der erfindungsgemäßen Vorrichtung mit hoher Effizienz eingesetzte Lichtenergie bedeutet, dass zum Zerkleinern bestimmter wasserhaltiger Steine weniger Lichtenergie und damit auch eine geringere Lichtleistung benötigt wird. Damit ist die Gefährdung von hinter oder neben den zu zerkleinernden Steinen liegenden Geweben, wie beispielsweise einer Blasenwand, bereits erheblich reduziert. Eine weitere Gefahrenreduktion tritt dadurch auf, dass die Fläche des Querschnitts des Hohlkegels mit der Entfernung zu der Applikationsfaser schnell anwächst und damit die Intensität des über diese Querschnittsfläche verteilten Laserlichts schnell abnimmt. In vergleichsweise kleinem Abstand zu der Applikationsfaser unterschreitet die Intensität des austretenden Laserlichts eine Intensitätsuntergrenze unterschritten, unter der keine Gefährdung von Geweben mehr auftritt, auf die das Laserlicht auftrifft. Die applizierte Lichtenergie löst den für das Zerkleinern der wasserhaltigen Steine genutzten Verdampfungsprozess des Wassers deshalb nur unmittelbar vor der Applikationsfaser aus. Damit sind ungewollte Gewebebeschädigungen sehr viel besser vermeidbar als bei Laserlicht, das über einen Vollkegel mit kleinem Vollwinkel verteilt austritt.

Vorzugsweise koppelt die Fokussieroptik den Teil des an dem Lichtausgabeort ausgegebenen Laserlichts unter einem Winkel zwischen seiner mittleren Lichtausbreitungsrichtung und der Faserachse im Bereich von 4° bis 9° in die Applikationsfaser.

In der alternativ oder zusätzlich implementierten anderen Ausführungsform der erfindungsgemäßen Vorrichtung ist der Winkel zwischen der mittleren Lichtausbreitungsrichtung des in die Applikationsfaser eingekoppelten Laserlichts und der Faserachse verstellbar, um den Kegelwinkel des Hohlkegels einzustellen, über den sich das aus der Applikationsfaser austretende Laserlicht verteilt. Der Winkel kann durch Verschwenken des Faseranschlusses stufenlos einstellbar sein. In Stufen kann der Winkel durch Verwenden verschiedener Applikationsfasern mit unterschiedlichen Neigungen zwischen ihren Lichteintrittsquerschnitten und ihren Faserachsen eingestellt werden. Der Winkel ist in einem Bereich von 0° und mindestens 5° und vorzugsweise in einem Bereich von 0° und mindestens 7° oder mindestens 10° einstellbar. Das aus der Applikationsfaser austretende Laserlicht kann dann entweder bei einem Winkel von 0° in einen Vollkegel mit sehr kleinem Kegelvollwinkel gerichtet werden oder mit zunehmendem Winkel in einen Hohlkegel mit größer werdendem und sich an den Aperturvollwinkel der Applikationsfaser annähernden Kegelvollwinkel. So kann beispielsweise Laserlicht in chirurgischen Anwendungen einmal mit hoher und stark lokalisierter Intensität zum Schneiden von Weichgewebe und einmal mit geringerer und räumlich verteilter Intensität zum Koagulieren von Blut verwendet werden.

Der Laseranschluss der erfindungsgemäßen Vorrichtung kann ein weiterer Faseranschluss für eine Laserfaser des als Faserlaser ausgebildeten Lasers oder eine das Laserlicht zuleitende optische Faser des Lasers sein.

Im Strahlengang des Laserlichts zwischen dem Laseranschluss und dem Faseranschluss kann ein dichroitischen Strahlteiler angeordnet sein, der Pumplicht von dem an dem Ausgabeort ausgegebenen Laserlicht weg zu einer Strahlfalle der Vorrichtung hin auskoppelt. Damit wird sichergestellt, dass nur das gewünschte Laserlicht in den Lichteintrittsquerschnitt der Applikationsfaser eingekoppelt wird. Vielfach ist es jedoch unerheblich, ob auch Pumplicht aus dem an den Laseranschluss angeschlossenen Laser mit in die Applikationsfaser gelangt.

Alternativ oder zusätzlich kann im Strahlengang des Laserlichts zwischen dem Laseranschluss und dem Faseranschluss ein Strahlteiler angeordnet sein, der einen prozentualen Anteil des an dem Ausgabeort ausgegebenen Laserlichts zu einem Lichtleistungssensor der Vorrichtung hin auskoppelt, um die Intensität des an dem Ausgabeort ausgegebenen Laserlichts zu erfassen und überwachen. Vor dem Lichtleistungsdetektor kann optional ein Diffusor oder eine Streuscheibe angeordnet sein, um das Signal des Lichtleistungsdetektors zu stabilisieren.

In einer Reflektionsrichtung des in den Lichteintrittsquerschnitt fokussierten Teils des Laserlichts, kann eine Strahlfalle der Vorrichtung angeordnet sein, um einen an dem Lichteintrittsquerschnitt der Applikationsfaser reflektierten Anteil des Laserlichts aufzufangen. Alternativ kann dort ein Reflektor angeordnet sein, der den reflektierten Anteil des Laserlichts, der typischerweise etwa vier Prozent des in den Lichteintrittsquerschnitt fokussierten Laserlichts ausmacht, zurück zu dem Lichteintrittsquerschnitt zu reflektieren. Ein solcher Reflektor kann beispielsweise einen Hohlspiegel oder eine Reflektorlinse und einen Reflektorspiegel aufweisen.

In einer Rückrichtung, die unter einem Winkel zu der Faserachse verläuft, welcher um mindestens 2° kleiner oder größer als der Winkel zwischen der mittleren Lichtausbreitungsrichtung des in den Lichteintrittsquerschnitt fokussierten Laserlichts und der Faserachse ist, kann ein weiterer Lichtleistungssensor der Vorrichtung angeordnet sein. Dieser weitere Lichtleistungsdetektor detektiert Licht, das in der Rückrichtung aus dem Lichteintrittsquerschnitt der Applikationsfaser austritt und das sich damit nicht in dem Hohlkegel befindet, welcher sich auch dort aus von einem perfekten Lichtaustrittsquerschnitt am distalen Ende der Applikationsfaser reflektiertem Laserlicht ausbildet. Solches Licht außerhalb des Hohlkegels ist ein Hinweis darauf, dass der Lichtaustrittsquerschnitt nicht mehr unversehrt, sondern verunreinigt oder angeraut ist, was auf einen Verbrauch der Applikationsfaser hinweist.

Weiterhin kann ein Pilotlaser der Vorrichtung so angeordnet sein, dass er sichtbares Pilotlaserlicht mit einer mittleren Ausbreitungsrichtung längs deren Faserachse in die Applikationsfaser einkoppelt. Dieses Pilotlaserlicht kann zum Ausrichten der Laserfaser gegenüber einem zu zerkleinernden Stein oder einem anderen mit dem Laserlicht zu beaufschlagenden Objekt verwendet werden, wenn das Objekt beispielsweise mit einem Endoskop oder einer Kamera für das sichtbare Pilotlaserlicht betrachtet wird. Zum Zerkleinern von wasserhaltigen Steinen, aber auch das zum Schneiden von Weichgewebe und Koagulieren von Blut eingesetztes Laserlicht weist häufig eine Wellenlänge im nicht sichtbaren Infrarotbereich auf.

Eine erfindungsgemäße Laserapparatur zum Applizieren von Laserlicht für chirurgische Anwendungen und insbesondere zum Zerkleinern von wasserhaltigen Steinen in Körperhöhlen weist einen Laser, der an einem Lichtausgabeort Laserlicht mit einer mittleren Lichtausbreitungsrichtung ausgibt, eine Applikationsfaser zum Applizieren des Laserlichts mit einem quer zu einer Faserachse verlaufenden Lichteintrittsquerschnitt und eine Fokussieroptik auf, die einen Teil des von dem Laser an dem Lichtausgabeort ausgegebenen Laserlichts durch deren Lichteintrittsquerschnitt in die Applikationsfaser einkoppelt und dazu den Teil des Laserlichts mit einem Konvergenzvollwinkel von nicht mehr als 2° in den Lichteintrittsquerschnitt der Applikationsfaser fokussiert.

Zudem koppelt die Fokussieroptik den Teil des an dem Lichtausgabeort ausgegebenen Laserlichts unter einem Winkel zwischen seiner mittleren Lichtausbreitungsrichtung und der Faserachse im Bereich von 2° bis 10° in die Applikationsfaser ein und/oder der Winkel zwischen der mittleren Lichtausbreitungsrichtung des in die Applikationsfaser eingekoppelten Laserlichts und der Faserachse ist in einem Bereich von 0° bis mindestens 5° verstellbar.

Vorzugsweise koppelt die Fokussieroptik den Teil des an dem Lichtausgabeort ausgegebenen Laserlichts unter einem Winkel zwischen seiner mittleren Lichtausbreitungsrichtung und der Faserachse im Bereich von 4° bis 9° in die Applikationsfaser ein. Alternativ oder zusätzlich kann der Winkel zwischen der mittleren Lichtausbreitungsrichtung des in die Applikationsfaser eingekoppelten Laserlichts und der Faserachse in einem Bereich von 0° und mindestens 7° oder mindestens 10° verstellbar sein.

Bei der erfindungsgemäßen Laserapparatur wird wie bei der Verwendung der erfindungsgemäßen Vorrichtung das Laserlicht nur über einen kleinen Teilbereich deren numerischen Apertur in die Applikationsfaser eingekoppelt, wobei in der Applikationsfaser eine Ringmode angeregt werden kann. Dies hat zur Folge, dass sich das aus der Applikationsfaser an deren distalen Ende austretende Laserlicht auf einen Vollkegel mit sehr kleinem Kegelwinkel oder einen Hohlkegel mit nur kleinem Unterschied zwischen den Vollwinkeln der ihn begrenzenden äußeren und inneren Kegelmänteln konzentriert.

Der Laser der erfindungsgemäßen Laserapparatur ist vorzugsweise ein Thulium-Laser, der Laserlicht mit einer Wellenlänge von 1.940 nm abgibt, die von Wasser besonders stark absorbiert wird. Weiterhin ist der Laser vorzugsweise ein Faserlaser, der nur einen kleinen Divergenzvollwinkel des an dem Lichtausgabeort ausgegebenen Laserlichts aufweist, so dass der Konvergenzvollwinkel des in den Lichteintrittsquerschnitt der Applikationsfaser fokussierten Laserlichts von nicht mehr als 2° besonders leicht eingehalten werden kann. Insbesondere mit einem Thulium-Faserlaser kann das an dem Lichtausgabeort ausgegebene Laserlicht einen Strahldurchmesser von nicht mehr als 100 µm, vorzugsweise von nicht mehr als 50 µm und insbesondere von nicht mehr als 15 µm und einen Divergenzvollwinkel von nicht mehr als 20°, insbesondere von nicht mehr als 15° aufweisen.

Ein Divergenzvollwinkel von nicht mehr als 20° entspricht einer numerischen Apertur des Lasers von nicht mehr als NA=0,175, und ein Divergenzvollwinkel von nicht mehr als 15° entspricht einer numerischen Apertur des Lasers von nicht mehr als NA=0,13.

Der Strahldurchmesser des Laserlichts an dem Lichtausgabeort des Lasers wird durch die notwendiger Weise große Brennweite einer Fokussierlinse der Fokussieroptik erheblich vergrößert. Um dennoch kleiner als der Lichteintrittsquerschnitt der Applikationsfaser zu bleiben, darf der Strahldurchmesser des Laserlichts an dem Lichtausgabeort des Lasers also nicht zu groß sein. Sonst geht Laserlicht zwischen dem Laser und der Applikationsfaser verloren.

Wie schon angesprochen wurde, ist die Applikationsfaser der erfindungsgemäßen Laserapparatur eine Multimode-Faser. Vorzugsweise ist ein Durchmesser des Lichteintrittsquerschnitts der Multimode-Faser nicht größer als 1 mm, insbesondere nicht größer als 0,5 mm. Er kann auch nur etwa 200 µm betragen. Eine numerische Apertur der Multimode-Faser liegt allgemein in einem Bereich zwischen 0,1 und 0,3, insbesondere in einem Bereich zwischen 0,20 und 0,25 und damit etwa bei NA = 0,22, was einem Aperturvollwinkel von 26° entspricht. Dieser Aperturvollwinkel wird bei der erfindungsgemäßen Vorrichtung aber nicht ausgenutzt. Es reicht daher auch eine Applikationsfaser mit einer kleineren Apertur aus.

Die Fokussieroptik der erfindungsgemäßen Laserapertur kann insbesondere die Fokussieroptik einer die Applikationsfaser an den Laser anschließenden erfindungsgemäßen Vorrichtung sein. Dies impliziert, dass die erfindungsgemäße Laserapparatur alle Merkmale der erfindungsgemäßen Vorrichtung aufweisen kann.

Vorteilhafte Weiterbildungen der Erfindung ergeben sich aus den Patentansprüchen, der Beschreibung und den Zeichnungen.

Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen.

Hinsichtlich des Offenbarungsgehalts - nicht des Schutzbereichs - der ursprünglichen Anmeldungsunterlagen und des Patents gilt Folgendes: Weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen, was aber nicht für die unabhängigen Patentansprüche des erteilten Patents gilt.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Laseranschluss die Rede ist, ist dies so zu verstehen, dass genau ein Laseranschluss, zwei Laseranschlüsse oder mehr Laseranschlüsse vorhanden sind. Die in den Patentansprüchen angeführten Merkmale können durch weitere Merkmale ergänzt werden oder die einzigen Merkmale sein, die der Gegenstand des jeweiligen Patentanspruchs aufweist.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: illustriert die Funktion einer ersten Ausführungsform der erfindungsgemäßen Vorrichtung und der erfindungsgemäßen Laserapparatur mit verstellbarem Winkel zwischen einer mittleren Lichtausbreitungsrichtung des in eine Applikationsfaser eingekoppelten Laserlichts und der Faserachse bei einem eingestellten Winkel von 0°.
- **Fig. 2**: illustriert die Funktion einer zweiten Ausführungsform der erfindungsgemäßen Vorrichtung und der erfindungsgemäßen Laserapparatur mit einem Winkel von 7° zwischen der mittleren Lichtausbreitungsrichtung des in die Applikationsfaser eingekoppelten Laserlichts und der Faserachse.
- **Fig. 3**: illustriert eine konkrete Ausgestaltung der erfindungsgemäßen Vorrichtung in der Ausführungsform gemäß Fig. 2.
- **Fig. 4**: zeigt eine Ausgestaltung der erfindungsgemäßen Laserapparatur mit einer weiteren Ausgestaltung der Vorrichtung in der Ausführungsform gemäß Fig. 2.
- **Fig. 5**: erläutert ein Detail von Fig. 4, betreffend eine Nutzung von etwa 99 % von fokussiertem Laserlicht durch erneutes Einkoppeln von Fresnel-Reflektion; und
- **Fig. 6**: erläutert ein weiteres Detail von Fig. 4 betreffend die Erfassung eines Verbrauchszustands einer Applikationsfaser.

### FIGUREN BESCHREIBUNG

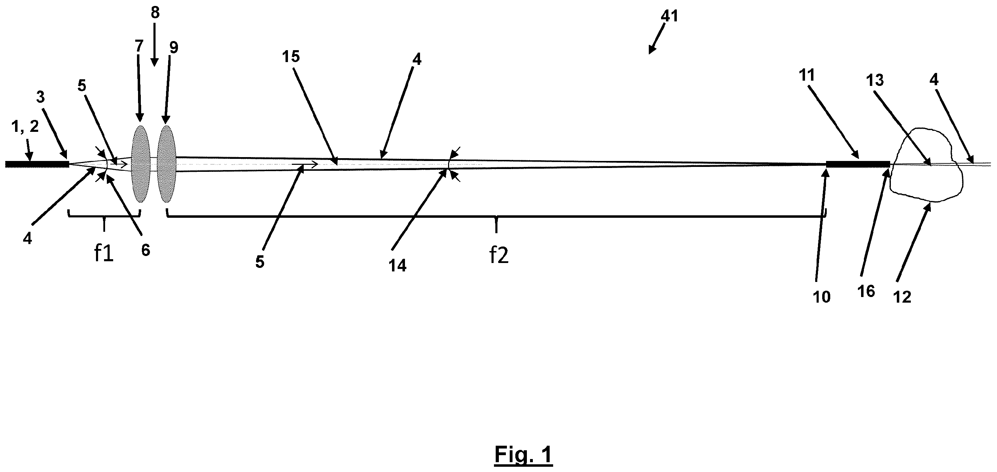

**Fig. 1** zeigt schematisch ein bei der erfindungsgemäßen Vorrichtung und der erfindungsgemäßen Laserapparatur zur Anwendung kommendes Konzept. Ein Laser 1, bei dem es sich um einen Thulium-Faserlaser 2 handelt, gibt an einem Lichtausgabeort 3 Laserlicht 4 mit einer mittleren Lichtausbreitungsrichtung 5, einen Divergenzvollwinkel 6 von 12,6°, was einer numerischen Apertur NA = 0,11 entspricht, einem Strahldurchmesser am Lichtausgabeort 3 von etwa 10 µm und einer Wellenlänge von 1.940 nm aus. Das Laserlicht 4 wird mit einer Kollimierlinse 7 einer Fokussieroptik 8 zunächst kollimiert und dann mit einer Fokussierlinse 9 der Fokussieroptik 8 fokussiert. Die Kollimierlinse 7 ist im Abstand ihrer Brennweite f1 zu dem Ausgabeort 3 angeordnet. Die Fokussierlinse 9 ist im Abstand ihrer Brennweite f2 zu einem Lichteintrittsquerschnitt 10 einer Applikationsfaser 11 zum Applizieren der Laserlichts 4 für chirurgische Anwendungen angeordnet. Der Lichteintrittsquerschnitt 10 verläuft hier normal zu einer Faserachse 15 der Applikationsfaser 11. Die Applikationsfaser 11 dient zum Applizieren des Laserlichts 4, d. h. zum Richten des Laserlichts 4 auf ein Objekt 12, wobei die Intensität des Laserlichts 4 auf ein Applikationsvolumen 13 konzentriert ist. Die Brennweite f2 der Fokussierlinse 9 ist zehnmal größer als die Brennweite f1 der Kollimierlinse 7. Dadurch ist ein Konvergenzvollwinkel 14 des in den Lichteintrittsquerschnitt 10 fokussierten Laserlichts 4 nur 1/10 so groß wie der Divergenzvollwinkel 6. Er beträgt also 1,3° was einer numerischen Apertur von NA = 0,01 entspricht. Die Applikationsfaser 11 weist eine typische numerische Apertur im Bereich von NA = 0,2, beispielsweise von NA = 0,22 auf. Das fokussierte Laserlicht 4 wird also über den Lichteintrittsquerschnitt 10 nur unter Ausnutzung eines kleinen Teils der numerischen Apertur der Applikationsfaser 11 in die Applikationsfaser 11 eingekoppelt. Ein Strahldurchmesser des fokussierten Laserlichts 4 im Bereich des Lichteintrittsquerschnitts 10 ist aufgrund des Verhältnisses der Brennweiten f1 und f2 hingegen zehnmal so groß wie der Strahlendurchmesser am Lichtausgabeort 3, beträgt also etwa 100 µm. Der Strahldurchmesser des fokussierten Laserlichts 4 ist damit immer noch deutlich kleiner als der Durchmesser des Lichteintrittsquerschnitts 10 von beispielsweise 200 µm oder 500 µm. Das Applikationsvolumen 13, über das sich das Laserlicht 4 in den Bereich des Objekts 12 verteilt, ist klein, weil das Laserlicht 4 am distalen Ende 16 der Applikationsfaser 11 nur über die numerische Apertur NA = 0,01 verteilt austritt, über die hinweg es in die Applikationsfaser 11 eingekoppelt wurde. Entsprechend ist die Lichtintensität des Laserlichts 4 in dem Applikationsvolumen 13 gegenüber einer Ausnutzung der vollen oder auch nur der halben numerischen Apertur der Applikationsfaser 11 stark erhöht. Wenn das Laserlicht 4 aus der Applikationsfaser 11 statt über eine numerische Apertur NA = 0,1 nur über die numerische Apertur NA = 0,01 verteilt austritt entspricht dies einer Steigerung der Intensität des Laserlichts 4 in dem Applikationsvolumen 13 um einen Faktor von etwa 100.

Bei der Vorrichtung gemäß Fig. 1 ist ein Winkel zwischen der mittleren Ausbreitungsrichtung 5 des in den Lichteintrittsquerschnitts 10 der Applikationsfaser 11 fokussierten und in die Laserfaser eingekoppelten Laserlichts 4 und der Faserachse 15 verstellbar, aber auf 0° eingestellt, so dass die Faserachse 15 und die Lichtausbreitungsrichtung 5 des fokussierten Laserlichts 4 zusammenfallen.

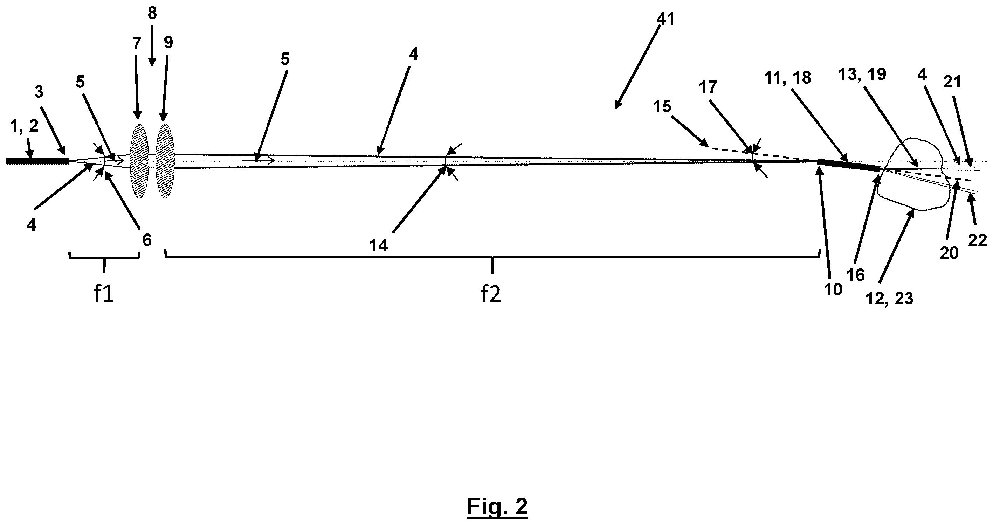

Die Vorrichtung gemäß **Fig. 2** unterscheidet sich von derjenigen gemäß Fig. 1 dadurch, dass die Faserachse 15 und die Lichtausbreitungsrichtung 5 des fokussierten Laserlichts 4 nicht zusammenfallen, sondern die mittlere Ausbreitungsrichtung 5 des in den Lichteintrittsquerschnitts 10 der Applikationsfaser 11 fokussierten und in die Laserfaser eingekoppelten Laserlichts 4 einen Winkel 17 von 7° zu der Faserachse 15 aufweist. Dies führt dazu, dass in der als Multimode-Faser 18 ausgebildeten Applikationsfaser 11 eine Ringmode angeregt wird, die auch als Donut-Mode bezeichnet wird. Dadurch tritt das Laserlicht 4 im Bereich des Objekts 12 in das Applikationsvolumen 13 hier in Form eines Hohlkegels 19 aus. Eine Kegelachse 20 des Hohlkegels 19 verläuft dabei koaxial zu der Faserachse 15 am distalen Ende 16 der Applikationsfaser 11. Der Hohlkegel 19 wird durch einen äußeren Kegelmantel 21 und einen inneren Kegelmantel 22 begrenzt. Ein Vollwinkel des äußeren Kegelmantels 21 ist gleich dem zweifachen des Winkels 17 plus dem halben Konvergenzwinkel 14, während ein Vollwinkel des inneren Kegelmantels 22 gleich dem doppelten des Winkels 17 minus dem halben Konvergenzwinkel 14 ist. In der Ausführungsform gemäß Fig. 2 wird das Laserlicht 4 in dem Applikationsvolumen 13 auf eine geringere Intensität konzentriert als in der Ausführungsform gemäß Fig. 1. Die Form des Applikationsvolumens 13 in Form des Hohlkegels 19 erweist sich jedoch als sehr effektiv beispielsweise beim Zerkleinern von Objekten 12 in Form von wasserhaltigen Steinen 23. Das Volumen des Hohlkegels 19 ist verglichen mit einem der vollen numerischen Apertur der Applikationsfaser 11 von NA = 0,22 entsprechenden Vollkegel um etwa einen Faktor 10 kleiner.

Im Vergleich zu Laserlicht eines Holmium:YAG-Lasers von 2.100 nm Wellenlänge ergibt sich zudem eine höhere Absorption durch Wasser, die das erhitzte Volumen um einen weiteren Faktor 2 reduziert. Dies bedeutet, dass sich eine Peak-Leistung eines Pulses des Laserlichts bei dem Laserlicht 4 von dem Thulium-Faserlaser 2 über ein zwanzigmal kleineres effektives Applikationsvolumen 13 verteilt als im Fall eines Holmium:YAG-Lasers. Dieselbe Lichtintensität wird also mit einer sehr viel geringeren Pulsenergie erreicht.

Zudem ergeben sich offenbar erhebliche zusätzliche Vorteile durch die Form des Hohlkegels 19 des Applikationsvolumens 13. Jedenfalls wird mit einer Anordnung gemäß Fig. 2 eine Zerkleinerungsrate von künstlichen Nierensteinen in Form sogenannter Begosteine beim Zerstäuben von 4,5 mg/s erreicht. Dies ist deutlich mehr als bislang beim Zerkleinern von Nierensteinen durch Zerstäuben mit Hilfe eines Thulium-Faserlasers mit maximal 3 mg/s erreicht wurde. Die Anordnung gemäß Fig. 2 ist auch für das Fragmentieren von Nierensteinen geeignet und erreicht dabei mit 7,5 mg/s sogar bessere Zerkleinerungsraten als der hierfür bislang übliche Holmium:YAG-Laser mit 6,6 mg/s. Diese Zahlenwerte basieren auf Thulium-Faserlasern gleicher Spitzenleistung und einem Holmium:YAG-Laser mit zwanzigmal höheren Spitzenleistung als derjenigen der Thulium-Faserlaser.

Da die hohe Zerkleinerungsrate bei der erfindungsgemäßen Vorrichtung mit einer relativ kleinen Lichtleistung des eingesetzten Laserlichts erreicht wird, ist die Gefahr von ungewollten Gewebebeschädigungen im Umfeld von zu zerkleinernden wasserhaltigen Steinen in Körperhöhlen bereits erheblich reduziert. Eine weitere Reduktion des Gefährdungspotentials ergibt sich dadurch, dass die Intensität des Laserlichts mit größer werdendem Querschnitt des Hohlkegels schnell abnimmt.

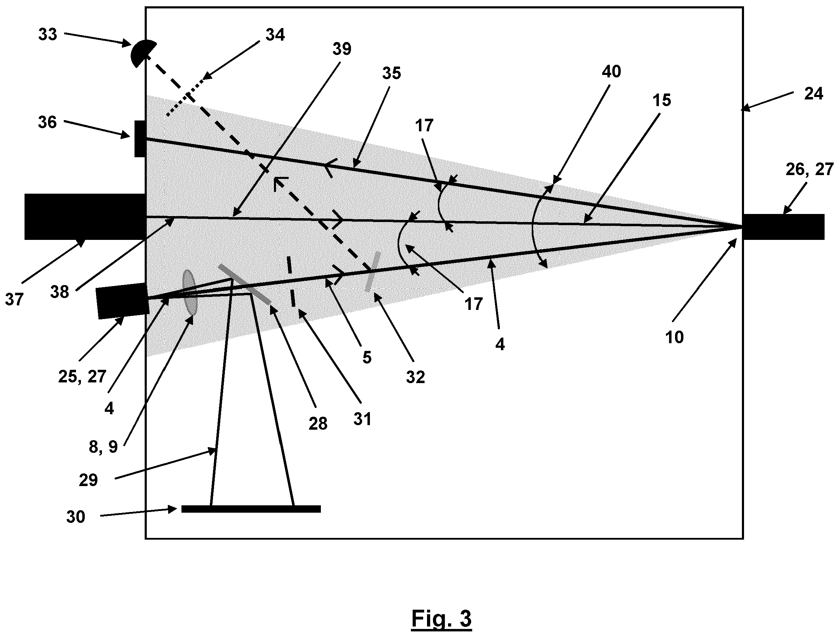

**Fig. 3** illustriert eine erfindungsgemäße Vorrichtung 24 zum Anschließen der Applikationsfaser 11 an den Laser 1 in der Anordnung gemäß Fig. 2. Für den Laser 1 bzw. den Thulium-Faserlaser 2 ist ein Laseranschluss 25 und für die Applikationsfaser 11 ein Faseranschluss 26 vorgesehen. Dabei kann der Faseranschluss 26 verschwenkbar sein, um den Winkel 17 zu variieren. Sowohl der Laseranschluss 25 als auch der Faseranschluss 26 kann ein Steckverbinder 27 für einen nicht separat dargestellten Faserstecker sein, in dem die jeweilige optische Faser verklebt oder anderweitig befestigt ist. Die Fokussieroptik 8 weist hier nur die Fokussierlinse 9 auf, d. h. dass an dem Lichtausgabeort 3 in die Vorrichtung 24 eintretende Laserlicht 4 wird nicht zuerst kollimiert und dann in den Lichteintrittsquerschnitt 10 fokussiert, sondern beides geschieht in einem Schritt.

Ein zwischen dem Laseranschluss 25 und dem Faseranschluss 26 angeordneter dichroitischer Strahlteiler 28 koppelt Pumplicht 29, das zusammen mit dem Laserlicht 4 in die Vorrichtung 24 eintritt, zu einer Strahlfalle 30 hin aus. Eine Aperturblende 31 begrenzt den Strahldurchmesser des fokussierten Laserlichts 4. Ein Strahlteiler 32 koppelt einen prozentualen Anteil des in die Vorrichtung 24 eintretenden Laserlichts 4 zu einem Lichtleistungsdetektor 33 hin aus, vor dem eine Streuscheibe 34 angeordnet ist, um das Signal des Lichtleistungsdetektors 33 beispielsweise im Hinblick auf die Ausbildung von Specklemustern stabilisiert. An dem Lichteintrittsquerschnitt 10 reflektiert die Applikationsfaser 11 einen Teil des fokussierten Laserlichts 4 von etwa 4 % in eine Reflektionsrichtung 35, die hier in Bezug auf die Faserachse 15 symmetrisch zu der mittleren Lichtausbreitungsrichtung 5 des fokussierten Laserlichts 4 verläuft. In der Ausführungsform gemäß Fig. 3 gelangt dieses reflektierte Laserlicht in eine Strahlfalle 36. Ein Pilotlaser 37 ist so angeordnet, dass er sichtbares Pilotlaserlicht 38 mit einer mittleren Lichtausbreitungsrichtung 39 längs der Faserachse 15 in die an den Faseranschluss 26 angeschlossene Applikationsfaser 11 einkoppelt. Weiter ist in Fig. 3 ein Aperturvollwinkel 40 der Applikationsfaser 11 eingezeichnet, der einer numerischen Apertur NA = 0,22 entspricht. Hieraus wird deutlich, wie wenig bei der erfindungsgemäßen Einkopplung des fokussierten Laserlichts 4 in den Lichteintrittsquerschnitt 10 von der vollen numerischen Apertur der Applikationsfaser 11 Gebrauch gemacht wird.

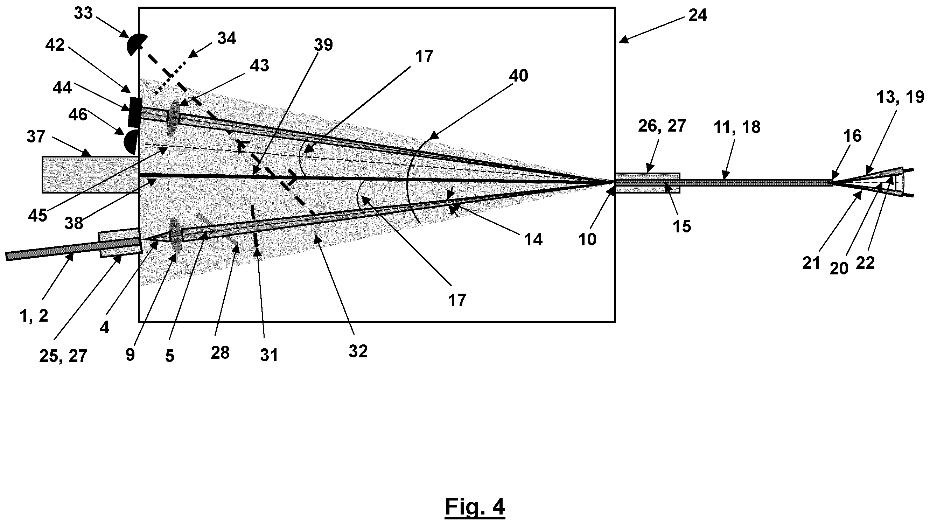

**Fig. 4** zeigt eine gegenüber Fig. 3 abgewandelte Ausführungsform der Vorrichtung 24 als Teil einer erfindungsgemäßen Laserapparatur 41. Dabei weist die Vorrichtung 24 gegenüber Fig. 3 folgende Abwandlungen auf. Statt der Strahlfalle 36 ist ein Reflektor 42 für das in die Reflektionsrichtung 35 reflektierte Laserlicht 4 vorgesehen, der das reflektierte Laserlicht 4 in den Lichteintrittsquerschnitt 10 zurückwirft. Der Reflektor 42 weist hier eine Reflektorlinse 43 und einen Reflektorspiegel 44 auf. Weiterhin ist in einer Rückrichtung 45, die unter einem anderen Winkel als dem Winkel 17 zu der Faserachse 15 verläuft, ein weiterer Lichtleistungsdetektor 46 angeordnet.

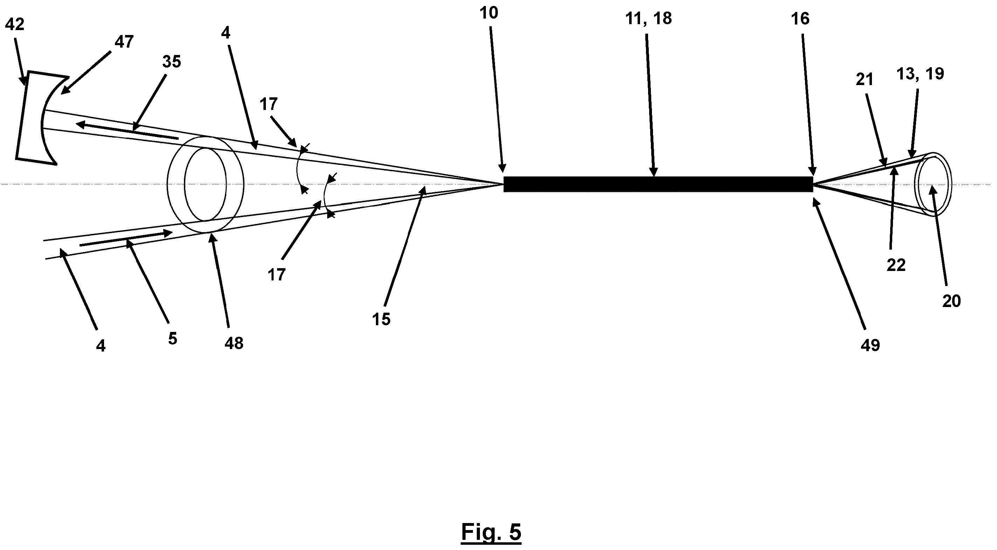

**Fig. 5** erläutert die Funktion des Reflektors 42 gemäß Fig. 4 der hier als Hohlspiegel 47 ausgebildet ist. An dem Lichteintrittsquerschnitt 10 von der Applikationsfaser 11 reflektiertes Laserlicht 4 wird von dem Reflektor 42 erneut unter dem Winkel 17 zu der Faserachse 15 in den Lichteintrittsquerschnitt 10 reflektiert. Dadurch werden 99 % des in den Lichteintrittsquerschnitt 10 fokussierten Laserlichts 4 tatsächlich in die Applikationsfaser 11 eingekoppelt. Weiterhin deutet Fig. 5 an, dass ein Bild 48 einer Rückreflektion an einem Lichtaustrittsquerschnitt 49 an dem distalen Ende 16 der Applikationsfaser 11 ring- oder donutförmig ist, solange der Lichtaustrittsquerschnitt 49 der Applikationsfaser 11 sauber und glatt ist sowie normal zu der Faserachse 15 verläuft.

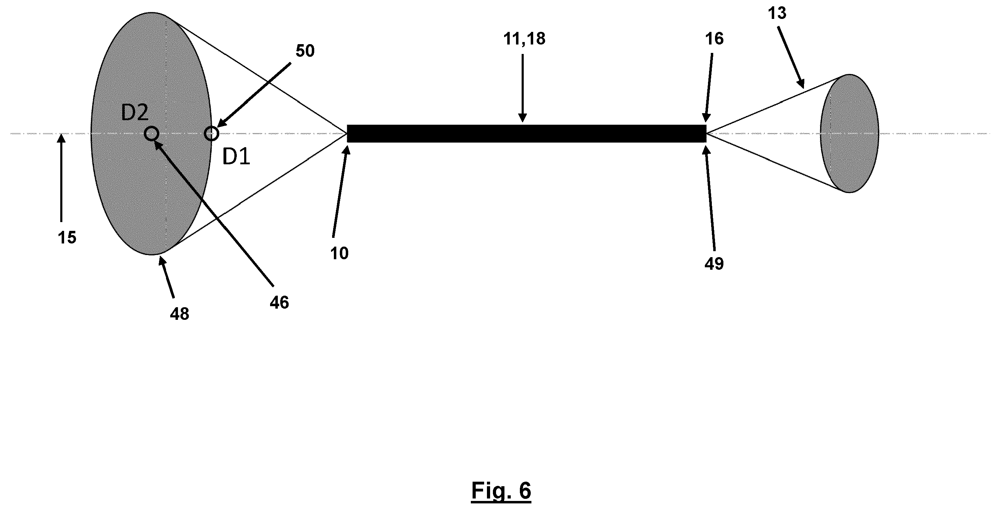

Hiervon macht der in Fig. 4 eingezeichnete Lichtleistungsdetektor 46 Gebrauch. Wenn gemäß **Fig. 6** das Bild 48 kein Ring, sondern eine Kreisscheibe ist, weist dies auf eine Aufrauhung oder eine sonstige Beeinträchtigung des Lichtaustrittsquerschnitt 49 der Applikationsfaser 11 an dem distalen Ende 16 hin. Diese Aufrauhung führt auch dazu, dass das Applikationsvolumen 13 nicht mehr die Form des Hohlkegels 19 aufweist, sondern vollkegelförmig wird. Noch sicherer als allein aus dem Signal des Lichtleistungsdetektors 46 nahe der Faserachse 15 kann die Aufrauhung des Lichtaustrittsquerschnitts 49 durch die Differenz des Signals des Lichtleistungssensors 46 zu einem weiteren in Fig. 4 nicht dargestellten Lichtleistungssensor 50 erfasst werden, der im Bereich des ringförmigen Bilds 48 der Rückreflektion gemäß Fig. 5 angeordnet ist. Bei dem ringförmigen Bild 48 ist die Differenz der Signale der beiden Lichtleistungssensoren 46 und 50 maximal. Mit zunehmender Aufrauhung des Lichtaustrittsquerschnitts 49 nimmt die Differenz ab. Wenn sie unter einen bestimmten Wert fällt, ist es Zeit, die Applikationsfaser 11 auszutauschen.

### BEZUGSZEICHENLISTE

- 1: Laser
- 2: Thulium-Faserlaser
- 3: Lichtausgabeort
- 4: Laserlicht
- 5: mittlere Lichtausbreitungsrichtung
- 6: Divergenzvollwinkel
- 7: Kollimierlinse
- 8: Fokussieroptik
- 9: Fokussierlinse
- 10: Lichteintrittsquerschnitt
- 11: Applikationsfaser
- 12: Objekt
- 13: Applikationsvolumen
- 14: Konvergenzwinkel
- 15: Faserachse
- 16: distales Ende
- 17: Winkel
- 18: Multimode-Faser
- 19: Hohlkegel
- 20: Kegelachse
- 21: äußerer Kegelmantel
- 22: innerer Kegelmantel
- 23: wasserhaltiger Stein
- 24: Vorrichtung
- 25: Laseranschluss
- 26: Faseranschluss
- 27: Steckverbinder
- 28: dichroitischer Strahlteiler
- 29: Pumplicht
- 30: Strahlfalle
- 31: Aperturblende
- 32: prozentualer Strahlteiler
- 33: Lichtleistungssensor
- 34: Streuscheibe
- 35: Reflektionsrichtung
- 36: Strahlfalle
- 37: Pilotlaser
- 38: Pilotlaserlicht
- 39: mittlere Ausbreitungsrichtung des Pilotlaserlichts 38
- 40: Aperturvollwinkel
- 41: Laserapparatur
- 42: Reflektor
- 43: Reflektorlinse
- 44: Reflektorspiegel
- 45: Rückrichtung
- 46: Lichtleistungsdetektor
- 47: Hohlspiegel
- 48: Bild
- 49: Lichtaustrittsquerschnitt
- 50: Lichtleistungsdetektor

## Patentansprüche

1. Vorrichtung (24) zum Anschließen einer Applikationsfaser (11) zum Applizieren von Laserlicht (4) für chirurgische Anwendungen, die einen quer zu einer Faserachse (15) verlaufenden Lichteintrittsquerschnitt (10) aufweist, an einen Laser (1), der das Laserlicht (4) an einem Lichtausgabeort (3) mit einer mittleren Lichtausbreitungsrichtung (5) ausgibt,
- mit einem Laseranschluss (25) zum Anschließen des Lasers (1), wobei der Laseranschluss (25) den Lichtausgabeort (3) und die mittlere Lichtausbreitungsrichtung (5) des Laserlichts (4) gegenüber der Vorrichtung (24) ausrichtet,
- mit einem Faseranschluss (26) zum Anschließen der Applikationsfaser (11), wobei der Faseranschluss (26) die Faserachse (15) und den Lichteintrittsquerschnitt (10) der Applikationsfaser (11) gegenüber der Vorrichtung (24) ausrichtet, und
- mit einer derart relativ zu dem Laseranschluss (25) und dem Faseranschluss (26) angeordneten Fokussieroptik (8), dass die Fokussieroptik (8) einen Teil des an dem Lichtausgabeort (3) ausgegebenen Laserlichts (4) durch deren Lichteintrittsquerschnitt (10) in die an den Faseranschluss (26) angeschlossene Applikationsfaser einkoppelt,
**dadurch gekennzeichnet,**
- **dass** die Fokussieroptik (8) den Teil des Laserlichts (4) mit einem Konvergenzvollwinkel (14) von nicht mehr als 2° in den Lichteintrittsquerschnitt (10) fokussiert und
- **dass**
die Fokussieroptik (8) den Teil des an dem Lichtausgabeort (3) ausgegebenen Laserlichts (4) unter einem Winkel (17) zwischen der mittleren Lichtausbreitungsrichtung (5) des in die Applikationsfaser (11) eingekoppelten Laserlichts (4) und der Faserachse (15) im Bereich von 2° bis 10° in die Applikationsfaser (11) einkoppelt und/oder
der Faseranschluss (26) derart verschwenkbar ist, dass der Winkel (17) zwischen der mittleren Lichtausbreitungsrichtung (5) des in die Applikationsfaser (11) eingekoppelten Laserlichts (4) und der Faserachse (15) in einem Bereich von 0° bis mindestens 5° verstellbar ist.

2. Vorrichtung (24) nach Anspruch 1, wobei die Fokussieroptik (8) eine Kollimierlinse (7) und eine Fokussierlinse (9) aufweist, wobei die Fokussierlinse (9) eine um mindestens einen Faktor 5, vorzugsweise um mindestens einen Faktor 8 größere Brennweite (f2) aufweist als die Kollimierlinse (7).

3. Vorrichtung (24) nach Anspruch 1 oder 2, wobei die Fokussieroptik (8) eine Fokussierlinse (9) mit einer Brennweite (f2) von mindestens 10 cm, vorzugsweise mit einer Brennweite (f2) im Bereich zwischen 12 cm und 20 cm, aufweist.

4. Vorrichtung (24) nach einem der vorhergehenden Ansprüche, wobei die Fokussieroptik (8) den Teil des an dem Lichtausgabeort (3) ausgegebenen Laserlichts (4) unter dem Winkel (17) zwischen seiner mittleren Lichtausbreitungsrichtung (5) und der Faserachse (15) im Bereich von 4° bis 9° in die Applikationsfaser (11) einkoppelt.

5. Vorrichtung (24) nach einem der vorhergehenden Ansprüche, wobei der Faseranschluss (26) derart verschwenkbar ist, dass der Winkel (17) zwischen der mittleren Lichtausbreitungsrichtung (5) des in die Applikationsfaser (11) eingekoppelten Laserlichts (4) und der Faserachse (15) in einem Bereich von 0° bis mindestens 7° oder von 0° bis mindestens 10° verstellbar ist.

6. Vorrichtung (24) nach einem der vorhergehenden Ansprüche, wobei im Strahlengang des Laserlichts (4) zwischen dem Laseranschluss (25) und dem Faseranschluss (26) ein dichroitischer Strahlteiler (28) angeordnet ist, der Pumplicht (29) von dem an dem Lichtausgabeort (3) ausgegebenen Laserlicht (4) weg zu einer Strahlfalle (30) der Vorrichtung (24) hin auskoppelt.

7. Vorrichtung (24) nach einem der vorhergehenden Ansprüche, wobei im Strahlengang des Laserlichts (4) zwischen dem Laseranschluss (25) und dem Faseranschluss (26) ein Strahlteiler (32) angeordnet ist, der einen prozentualen Anteil des an dem Lichtausgabeort (3) ausgegebenen Laserlichts (4) zu einem Lichtleistungsdetektor (33) der Vorrichtung (24) hin auskoppelt, wobei vor dem Lichtleistungsdetektor (33) optional ein Diffusor oder eine Streuscheibe (34) angeordnet ist.

8. Vorrichtung (24) nach einem der vorhergehenden Ansprüche, wobei in einer Reflektionsrichtung (35) des in den Lichteintrittsquerschnitt (10) fokussierten Teils des Laserlichts (4)
- eine Strahlfalle (36) der Vorrichtung (24) oder
- ein Reflektor (42) der Vorrichtung (24) angeordnet ist, wobei der Reflektor optional (42) einen Hohlspiegel (47) oder eine Reflektorlinse (43) und einen Reflektorspiegel (44) aufweist.

9. Vorrichtung (24) nach einem der vorhergehenden Ansprüche, wobei in einer Rückrichtung (45), die unter einem Winkel zu der Faserachse (15) verläuft, welcher um mindestens 2° kleiner oder größer als der Winkel (17) zwischen der mittleren Lichtausbreitungsrichtung (5) des in den Lichteintrittsquerschnitt (10) fokussierten Laserlichts (4) und der Faserachse (15) ist, ein weiterer Lichtleistungsdetektor (46) der Vorrichtung (24) angeordnet ist.

10. Vorrichtung (24) nach einem der vorhergehenden Ansprüche, wobei ein Pilotlaser (37) der Vorrichtung (24) so angeordnet ist, dass er sichtbares Pilotlaserlicht (38) mit einer mittleren Lichtausbreitungsrichtung (39) längs der Faserachse (15) in die Applikationsfaser (11) einkoppelt.

11. Laserapparatur für chirurgische Anwendungen mit
- einem Laser (1), der an einem Lichtausgabeort (3) Laserlicht (4) mit einer mittleren Lichtausbreitungsrichtung (5) ausgibt,
- einer Applikationsfaser (11) zum Applizieren des Laserlichts (4) mit einem quer zu einer Faserachse (15) verlaufenden Lichteintrittsquerschnitt, und
- einer Fokussieroptik (8), die einen Teil des von dem Laser (1) an dem Lichtausgabeort (3) ausgegebenen Laserlichts (4) durch deren Lichteintrittsquerschnitt (10) in die Applikationsfaser (11) einkoppelt,
**dadurch gekennzeichnet,**
- **dass** die Fokussieroptik (8) den Teil des Laserlichts (4) mit einem Konvergenzvollwinkel (14) von nicht mehr als 2° in den Lichteintrittsquerschnitt (10) der Applikationsfaser (11) fokussiert und
- **dass**
die Fokussieroptik (8) den Teil des an dem Lichtausgabeort (3) ausgegebenen Laserlichts (4) unter einem Winkel (17) zwischen der mittleren Lichtausbreitungsrichtung (5) des in die Applikationsfaser (11) eingekoppelten Laserlichts (4) und der Faserachse (15) im Bereich von 2° bis 10° in die Applikationsfaser (11) einkoppelt und/oder
der Winkel (17) zwischen der mittleren Lichtausbreitungsrichtung (5) des in die Applikationsfaser (11) eingekoppelten Laserlichts (4) und der Faserachse (15) in einem Bereich von 0° bis mindestens 5° verstellbar ist.

12. Laserapparatur nach Anspruch 11, wobei die Fokussieroptik (8) den Teil des an dem Lichtausgabeort (3) ausgegebenen Laserlichts (4) unter einem Winkel (17) zwischen seiner mittleren Lichtausbreitungsrichtung (5) und der Faserachse (15) im Bereich von 4° bis 9° in die Applikationsfaser (11) einkoppelt und/oder
- und wobei der Winkel (17) zwischen der mittleren Lichtausbreitungsrichtung (5) des in die Applikationsfaser (11) eingekoppelten Laserlichts (4) und der Faserachse (15) in einem Bereich von 0° bis mindestens 7° oder von 0° bis mindestens 10° verstellbar ist.

13. Laserapparatur nach einem der Ansprüche 11 und 12, wobei
der Laser (1) ein Thulium-Laser und/oder ein Faserlaser ist, bei dem das an dem Lichtausgabeort (3) ausgegebene Laserlicht (4) einen Strahldurchmesser von nicht mehr als 50 µm und einen Divergenzvollwinkel (6) von nicht mehr als 20° aufweist, wobei das an dem Lichtausgabeort (3) ausgegebene Laserlicht (4) optional einen Strahldurchmesser von nicht mehr als 15 µm und/oder einen Divergenzvollwinkel (6) von nicht mehr als 15° aufweist.

14. Laserapparatur nach einem der Ansprüche 11 bis 13, wobei
die Applikationsfaser (11) eine Multimode-Faser (18) ist, wobei ein Durchmesser des Lichteintrittsquerschnitts (10) nicht größer als 1 mm ist und wobei eine numerische Apertur der Multimode-Faser (18) in einem Bereich zwischen 0,1 und 0,3 liegt, wobei optional der Durchmesser des Lichteintrittsquerschnitts (10) nicht größer als 0,5 mm ist und/oder die numerische Apertur der Multimode-Faser (18) in einem Bereich zwischen 0,20 und 0,25 liegt.

15. Laserapparatur nach einem der Ansprüche 11 bis 14 mit einer die Applikationsfaser (11) an den Laser (1) anschließenden und die Fokussieroptik (8) umfassenden Vorrichtung (24) nach einem der Ansprüche 1 bis 10.

## Claims

1. A device (24) for connecting an application fibre (11), for applying laser light (4) for surgical applications, to a laser (1), the application fibre (11) having a light entry cross section (10) running crosswise to a fibre axis (15), the laser (1) outputting the laser light (4) at a light output location (3) with a mean light propagation direction (5), the device comprising
- a laser port (25) for connecting the laser (1), wherein the laser port (25) orientates the light output location (3) and the mean light propagation direction (5) of the laser light (4) with regard to the device (24),
- a fibre port (26) for connecting the application fibre (11), wherein the fibre port (26) orientates the fibre axis (15) and the light entry cross section (10) of the application fibre (11) with regard to the device (24), and
- a focusing optic (8) which is arranged with respect to the laser port (25) and the fibre port (26) in such a way that the focusing optic (8) couples a part of the laser light (4) output at the light output location (3) through the light entry cross section (10) of the application fibre into the application fibre connected to the fibre port (26),
**characterized in**
- **that** the focusing optic (8) focuses the part of the laser light (4) with a full convergence angle (14) of not more than 2° into the light entry cross section (10), and
- **that**
the focusing optic (8) couples the part of the laser light (4) output at the light output location (3) into the application fibre (11), at an angle (17) between the mean light propagation direction (5) of the laser light (4) coupled into the application fibre (11) and the fibre axis (15) in a range from 2° to 10°, and/or
the fibre port (26) is tiltable in such a way that the angle (17) between the mean light propagation direction (5) of the laser light (4) coupled into the application fibre (11) and the fibre axis (15) is adjustable within a range from 0° to at least 5°.

2. The device (24) of claim 1, wherein the focusing optic (8) comprises a collimating lens (7) and a focusing lens (9), wherein the focusing lens (9) has a focal length (f2) which is by a factor of at least 5, preferably by a factor of at least 8 longer than that one of the collimating lens (7).

3. The device (24) of claim 1 or 2, wherein the focusing optic (8) comprises a focusing lens (9) having a focal length (f2) of at least 10 cm, preferably having a focal length (f2) in a range between 12 cm and 20 cm.

4. The device (24) of any of the preceding claims, wherein the focusing optic (8) couples the part of the laser light (4) output at the light output location (3) at an angle (17) between its mean light propagation direction (5) and the fibre axis (15) in a range from 4° to 9° into the application fibre (11).

5. The device (24) of any of the preceding claims, wherein the fibre port (26) is tiltable such that the angle (17) between the mean light propagation direction (5) of the laser light (4) coupled into the application fibre (11) and the fibre axis (15) is adjustable in a range from 0° to at least 7° or from 0° to at least 10°.

6. The device (24) of any of the preceding claims, wherein a dichroic beam splitter (28), which couples-out pump light (29) away from the laser light (4) output at the light output location (3) towards a beam trap (30) of the device (24), is arranged in the beam path of the laser light (4) between the laser port (25) and the fibre port (26).

7. The device (24) of any of the preceding claims, wherein a beam splitter (32) is arranged in the beam path of the laser light (4) between the laser port (25) and the fibre port (26), the beam splitter coupling-out a percentage of the laser light (4) output at the light output location (3) towards a light power detector (33) of the device (24), wherein. optionally, a diffusor or a diffusion disc (34) is arranged in front of the light power detector (33).

8. The device (24) of any of the preceding claims, wherein, in a reflection direction (35) of the part of the laser light (4) focused into the light entry cross section (10),
- a beam trap (36) of the device (24) or
- a reflector (42) of the device (24)
is arranged, wherein the reflector (42) optionally comprises a hollow mirror (47) or a reflector lens (43) and a reflector mirror (44).

9. The device (24) of any of the preceding claims, wherein, in a reverse direction (45) which runs at an angle to the fibre axis (15) that is by at least 2° smaller or larger than the angle (17) between the mean light propagation direction (5) of the laser light (4) focused into the light entry cross section (10) and the fibre axis (15), a further light power detector (46) of the device (24) is arranged.

10. The device (24) of any of the preceding claims, wherein a pilot laser (37) of the device (24) is arranged such that it couples visible pilot laser light (38) with a mean light propagation direction (39) along the fibre axis (15) into the application fibre (11).

11. A laser apparatus for surgical applications, comprising
- a laser (1) which outputs laser light (4) with a mean light propagation direction (5) at a light output location (3),
- an application fibre (11) for applying the laser light (4), the application fibre (11) having a light entry cross section running perpendicular to the fibre axis (15), and
- a focusing optic (8) which couples a part of the laser light (4) output by the laser (1) at the light output location (3) through its light entry cross section (10) into the application fibre (11),
**characterized in**
- **that** the focusing optic (8) focuses the part of the laser light (4) with a full convergence angle (14) of not more than 2° into the light entry cross section (10) of the application fibre (11), and
- **that**
the focusing optic (8) couples the part of the laser light (4) output at the light output location (3) into the application fibre (11), at an angle (17) between the mean light propagation direction (5) of the laser light (4) coupled into the application fibre (11) and the fibre axis (15) in a range from 2° to 10°, and/or
the angle (17) between the mean light propagation direction (5) of the laser light (4) coupled into the application fibre (11) and the fibre axis (15) is adjustable within a range from 0° to at least 5°.

12. The laser apparatus of claim 11, wherein the focusing optic (8) couples the part of the laser light (4) output at the light output location (3) at an angle (17) between its mean light propagation direction (5) and the fibre axis (15) in a range from 4° to 9° into the application fibre (11), and/or wherein the angle (17) between the mean light propagation direction (5) of the laser light (4) coupled into the application fibre (11) and the fibre axis (15) is adjustable in a range from 0° to at least 7° or from 0° to at least 10°.

13. The laser apparatus of any of the claims 11 and 12, wherein the laser (1) is a thulium laser and/or a fibre laser in which the laser light (4) output at the light output location (3) has a beam diameter of not more than 50 µm and a full divergence angle (6) of not more than 20°, wherein the laser light (4) output at the light output location (3) optionally has a beam diameter of not more than 15 µm and/or a full divergence angle (6) of not more than 15°.

14. The laser apparatus of any of the claims 11 to 13, wherein the application fibre (11) is a multimode fibre (18), wherein a diameter of the light entry cross section (10) is not larger than 1 mm, and wherein a numerical aperture of the multimode fibre (18) is in a range between 0.1 and 0.3, wherein, optionally, the diameter of the light entry cross section (10) is not larger than 0.5 mm and/or that the numerical aperture of the multimode fibre (18) is in a range between 0.20 and 0.25.

15. The laser apparatus of any of the claims 11 to 14 comprising an device (24) of any of the claims 1 to 10 connecting the application fibre (11) to the laser (1) and including the focusing optic (8).

## Revendications

1. Dispositif (24) pour raccorder une fibre d'application (11) pour appliquer de la lumière laser (4) pour des applications chirurgicales, qui présente une section d'entrée de lumière (10) s'étendant transversalement à un axe de fibre (15), à un laser (1), qui émet la lumière laser (4) à un site d'émission lumineuse (3) avec une direction moyenne de propagation lumineuse (5),
- avec un raccordement laser (25) pour raccorder le laser (1), le raccordement laser (25) alignant le site d'émission lumineuse (3) et la direction moyenne de propagation lumineuse (5) de la lumière laser (4) par rapport au dispositif (24),
- avec un raccordement de fibre (26) pour raccorder la fibre d'application (11), le raccordement de fibre (26) alignant l'axe de fibre (15) et la section d'entrée de lumière (10) de la fibre d'application (11) par rapport au dispositif (24), et
- avec une optique de focalisation (8) disposée de manière relative au raccordement laser (25) et au raccordement de fibre (26) telle que l'optique de focalisation (8) couple une partie de la lumière laser (4) émise au site d'émission lumineuse (3) à travers sa section d'entrée de lumière (10) dans la fibre d'application raccordée au raccordement de fibre (26),
**caractérisé en ce que**
- l'optique de focalisation (8) focalise la partie de la lumière laser (4) avec un angle d'ouverture de convergence (14) ne dépassant pas 2° dans la section d'entrée de lumière (10) et
- que
l'optique de focalisation (8) couple la partie de la lumière laser (4) émise au site d'émission lumineuse (3) sous un angle (17) entre la direction moyenne de propagation lumineuse (5) de la lumière laser (4) couplée dans la fibre d'application (11) et l'axe de fibre (15) dans une plage de 2° à 10° dans la fibre d'application (11) et/ou
le raccordement de fibre (26) est orientable de telle sorte que l'angle (17) entre la direction moyenne de propagation lumineuse (5) de la lumière laser (4) couplée dans la fibre d'application (11) et l'axe de fibre (15) est réglable dans une plage de 0° à au moins 5°.

2. Dispositif (24) selon la revendication 1, où l'optique de focalisation (8) comprend une lentille de collimation (7) et une lentille de focalisation (9), la lentille de focalisation (9) présentant une distance focale (f2) supérieure d'au moins un facteur 5, de préférence d'au moins un facteur 8, à celle de la lentille de collimation (7).

3. Dispositif (24) selon la revendication 1 ou 2, où l'optique de focalisation (8) comprend une lentille de focalisation (9) avec une distance focale (f2) d'au moins 10 cm, de préférence avec une distance focale (f2) dans une plage comprise entre 12 cm et 20 cm.

4. Dispositif (24) selon l'une des revendications précédentes, où l'optique de focalisation (8) couple la partie de la lumière laser (4) émise au site d'émission lumineuse (3) sous l'angle (17) entre sa direction moyenne de propagation lumineuse (5) et l'axe de fibre (15) dans une plage de 4° à 9° dans la fibre d'application (11).

5. Dispositif (24) selon l'une des revendications précédentes, où le raccordement de fibre (26) est orientable de telle sorte que l'angle (17) entre la direction moyenne de propagation lumineuse (5) de la lumière laser (4) couplée dans la fibre d'application (11) et l'axe de fibre (15) est réglable dans une plage de 0° à au moins 7° ou de 0° à au moins 10°.

6. Dispositif (24) selon l'une des revendications précédentes, où dans le trajet du faisceau de la lumière laser (4) entre le raccordement laser (25) et le raccordement de fibre (26), un séparateur de faisceau dichroïque (28) est disposé, qui extrait la lumière de pompage (29) de la lumière laser (4) émise au site d'émission lumineuse (3) vers un piège à faisceau (30) du dispositif (24).

7. Dispositif (24) selon l'une des revendications précédentes, où dans le trajet du faisceau de la lumière laser (4) entre le raccordement laser (25) et le raccordement de fibre (26), un séparateur de faisceau (32) est disposé, qui extrait une fraction en pourcentage de la lumière laser (4) émise au site d'émission lumineuse (3) vers un détecteur de puissance lumineuse (33) du dispositif (24), un diffuseur ou une plaque de diffusion (34) étant disposé en option devant le détecteur de puissance lumineuse (33).

8. Dispositif (24) selon l'une des revendications précédentes, où dans une direction de réflexion (35) de la partie de la lumière laser (4) focalisée dans la section d'entrée de lumière (10)
- un piège à faisceau (36) du dispositif (24) ou
- un réflecteur (42) du dispositif (24)
est disposé, le réflecteur (42) présentant en option un miroir concave (47) ou une lentille de réflecteur (43) et un miroir de réflecteur (44).

9. Dispositif (24) selon l'une des revendications précédentes, où dans une direction opposée (45), qui s'étend sous un angle par rapport à l'axe de fibre (15) qui est d'au moins 2° inférieur ou supérieur à l'angle (17) entre la direction moyenne de propagation lumineuse (5) de la lumière laser (4) focalisée dans la section d'entrée de lumière (10) et l'axe de fibre (15), un autre détecteur de puissance lumineuse (46) du dispositif (24) est disposé.

10. Dispositif (24) selon l'une des revendications précédentes, où un laser pilote (37) du dispositif (24) est disposé de telle sorte qu'il couple une lumière de laser pilote (38) visible avec une direction moyenne de propagation lumineuse (39) le long de l'axe de fibre (15) dans la fibre d'application (11).

11. Appareillage laser pour des applications chirurgicales comprenant
- un laser (1), qui émet à un site d'émission lumineuse (3) une lumière laser (4) avec une direction moyenne de propagation lumineuse (5),
- une fibre d'application (11) pour appliquer la lumière laser (4) avec une section d'entrée de lumière s'étendant transversalement à un axe de fibre (15), et
- une optique de focalisation (8), qui couple une partie de la lumière laser (4) émise par le laser (1) au site d'émission lumineuse (3) à travers sa section d'entrée de lumière (10) dans la fibre d'application (11),
**caractérisé en ce que**
- l'optique de focalisation (8) focalise la partie de la lumière laser (4) avec un angle d'ouverture de convergence (14) ne dépassant pas 2° dans la section d'entrée de lumière (10) de la fibre d'application (11) et
- que
l'optique de focalisation (8) couple la partie de la lumière laser (4) émise au site d'émission lumineuse (3) sous un angle (17) entre la direction moyenne de propagation lumineuse (5) de la lumière laser (4) couplée dans la fibre d'application (11) et l'axe de fibre (15) dans une plage de 2° à 10° dans la fibre d'application (11) et/ou
l'angle (17) entre la direction moyenne de propagation lumineuse (5) de la lumière laser (4) couplée dans la fibre d'application (11) et l'axe de fibre (15) est réglable dans une plage de 0° à au moins 5°.

12. Appareillage laser selon la revendication 11, où l'optique de focalisation (8) couple la partie de la lumière laser (4) émise au site d'émission lumineuse (3) sous un angle (17) entre sa direction moyenne de propagation lumineuse (5) et l'axe de fibre (15) dans une plage de 4° à 9° dans la fibre d'application (11) et/ou où l'angle (17) entre la direction moyenne de propagation lumineuse (5) de la lumière laser (4) couplée dans la fibre d'application (11) et l'axe de fibre (15) est réglable dans une plage de 0° à au moins 7° ou de 0° à au moins 10°.

13. Appareillage laser selon l'une des revendications 11 et 12, où le laser (1) est un laser au thulium et/ou un laser à fibre, dans lequel la lumière laser (4) émise au site d'émission lumineuse (3) présente un diamètre du faisceau ne dépassant pas 50 µm et un angle d'ouverture de divergence (6) ne dépassant pas 20°, la lumière laser (4) émise au site d'émission lumineuse (3) présentant en option un diamètre du faisceau ne dépassant pas 15 µm et/ou un angle d'ouverture de divergence (6) ne dépassant pas 15°.

14. Appareillage laser selon l'une des revendications 11 à 13, où la fibre d'application (11) est une fibre multimode (18), un diamètre de la section d'entrée de lumière (10) n'étant pas supérieur à 1 mm et une ouverture numérique de la fibre multimode (18) se situant dans une plage comprise entre 0,1 et 0,3, le diamètre de la section d'entrée de lumière (10) n'étant en option pas supérieur à 0,5 mm et/ou l'ouverture numérique de la fibre multimode (18) se situant dans une plage comprise entre 0,20 et 0,25.

15. Appareillage laser selon l'une des revendications 11 à 14 avec un dispositif (24) raccordant la fibre d'application (11) au laser (1) et comprenant l'optique de focalisation (8) selon l'une des revendications 1 à 10.
